# EUROPEAN PATENT APPLICATION

(11) **EP 4 631 553 A1**
(43) Date of publication of application: **15.10.2025**
(21) Application number: 23899706.8
(22) Date of filing: 13.11.2023
(51) Int. Cl.: A61M 15/00, B65H 23/195

(54) **ROTATION STROKE COMPENSATION MECHANISM AND DRUG ADMINISTRATION DISPENSER**

(30) Priority: 09.12.2022 CN 202211586397
(71) Applicant: Transpire Bio Inc, Plantation, FL 33324 (US)
(72) Inventor: ZHANG, Xieen, Shenzhen, Guangdong 518102 (CN); OUYANG, Guanglin, Shenzhen, Guangdong 518102 (CN); LI, Lei, Shenzhen, Guangdong 518102 (CN); ZOU, Yujie, Shenzhen, Guangdong 518102 (CN); LIU, Yongwei, Shenzhen, Guangdong 518102 (CN)
(74) Representative: Latscha Schöllhorn Partner AG
(86) International application number: PCT/CN2023/131352
(87) International publication number: WO 2024/120126

(57) **Abstract**

This application provides a rotation compensation mechanism and a medicine delivery dispenser. The rotation compensation mechanism includes: an active rotation member, configured to be connected to a driving assembly and rotate under an action of the driving assembly; a driven rotation member, rotatably connected to the active rotation member and configured to wind a to-be-wound object; and a damping assembly, positioned between the active rotation member and the driven rotation member and configured to generate torque between the active rotation member and the driven rotation member, to enable the active rotation member to apply driving torque to the driven rotation member through the damping assembly. The driving torque is not greater than a preset threshold. The preset threshold is driving torque when the active rotation member rotates relative to the driven rotation member. The rotation compensation mechanism can perform a rotation compensation function.

## Description

### CROSS-REFERENCE TO RELATED APPLICATIONS

This application claims priority to Chinese Patent Application No. 202211586397.8, filed on December 9, 2022, which is incorporated herein by reference in its entirety.

### TECHNICAL FIELD

This application relates to the field of medical devices, and in particular, to a rotation compensation mechanism and a medicine delivery dispenser used in inhalers.

### BACKGROUND

In a typical powder-delivery inhaler device, a medicine in a form of a power or a tablet is loaded on an elongated blister strip that is overlayed with a cover strip, and the medicine can be provided to a patient after the cover strip of the blister strip is peeled. The cover strip is peeled, leaving two parts: a cover strip and a blister strip. The cover strip after being peeled is wound around a wind-up wheel and a stroke compensation mechanism controls the rate at which the wind-up wheel turns by compensating for an ever increasing diameter.

An elastic blade wheel or a torsion spring compensation wheel is conventionally used in the stroke compensation mechanism for the cover strip in common powder-delivery inhaler devices, but the two mechanisms have some defects. For example, in a powder-delivery inhaler devices with two medicine strips, because the rotation directions of the two cover strips are opposite, the two cover strips cannot share the same stroke compensation mechanism. Consequently, a fool-proof design and the like are required for the device.

### SUMMARY

In aspects described herein, a rotation compensation mechanism and a medicine delivery dispenser can reduce or resolve a technical problem for a powder-delivery inhaler device with two medicine strips. Specifically, the technical problem that arises because the rotation directions of two cover strips are opposite, meaning that the two cover strips cannot share the same stroke compensation mechanism. Consequently, it is necessary to make a fool-proof design for the powder aerosol delivery system.

To resolve the technical problem, a technical solution applied to this application is to provide a rotation compensation mechanism. The rotation compensation mechanism includes an active rotation member, configured to be connected to a driving assembly and rotate under an action of the driving assembly;
a driven rotation member, rotatably connected to the active rotation member and configured to wind a to-be-wound object; and
a damping assembly, positioned between the active rotation member and the driven rotation member and configured to generate torque between the active rotation member and the driven rotation member, to enable the active rotation member to apply driving torque to the driven rotation member through the damping assembly, where
the driving torque is not greater than a preset threshold; and the preset threshold is driving torque when the active rotation member rotates relative to the driven rotation member.

In an embodiment, the active rotation member applies same driving torque to the driven rotation member during clockwise rotation and counterclockwise rotation.

In an embodiment, the damping assembly does not include an elastic damper made of a rigid material.

In an embodiment, the damping assembly includes one or more of an elastic material damper, a viscous grease damper, and a magnetic damper.

In an embodiment, one of the active rotation member and the driven rotation member has a rotary shaft, the other has a hole, and the rotary shaft is inserted into the hole.

In an embodiment, the active rotation member includes a rotary shaft and a base plate; the rotary shaft is vertically connected to the side surface of the base plate; the driven rotation member includes a hollow columnar body having the hole, and is sleeved on the rotary shaft; and the damping assembly is positioned between the base plate and the end surface of the hollow columnar body, and/or is positioned between the outer surface of the rotary shaft and the inner surface of the hollow columnar body.

In an embodiment, the damping assembly includes a first elastic material damper, and the first elastic material damper is positioned between the base plate and the end surface of the hollow columnar body, and/or is positioned between the outer surface of the rotary shaft and the inner surface of the hollow columnar body; and the first elastic material damper is in the shape of a closed loop.

In an embodiment, the damping assembly further includes at least one second elastic material damper spaced apart; and the at least one second elastic material damper is positioned between the base plate and the end surface of the hollow columnar body and is located on the side of the first elastic material damper close to the rotary shaft; and/or the at least one second elastic material damper is positioned between the outer surface of the rotary shaft and the inner surface of the hollow columnar body and is located on the side of the first elastic material damper close to the base plate.

In an embodiment, the at least one second elastic material damper is in the shape of a closed loop, and the first elastic material damper and the at least one second elastic material damper are coaxially sleeved; or
the at least one second elastic material damper is in the sheet shape and is spaced apart in the circumferential direction of the rotary shaft to form at least one ring shape, and the first elastic material damper and the at least one ring shape are coaxially sleeved.

In an embodiment, torque generated between the active rotation member and the driven rotation member by the first elastic material damper is less than torque generated between the active rotation member and the driven rotation member by the at least one second elastic material damper.

In an embodiment, a plurality of second elastic material dampers are provided, and torque generated between the active rotation member and the driven rotation member by the plurality of second elastic material dampers gradually increases in the direction closer to the rotary shaft.

In an embodiment, the damping assembly includes the elastic material damper, a first engaging groove is provided on the side surface of the active rotation member facing the driven rotation member, and/or a second engaging groove is provided on the side surface of the driven rotation member facing the active rotation member; and
the elastic material damper is partially elastically compressed in the first engaging groove and abuts against the bottom wall of the first engaging groove; and/or the elastic material damper is partially elastically compressed in the second engaging groove and abuts against the bottom wall of the second engaging groove; and the driven rotation member is attached to the surface of the active rotation member.

In an embodiment, the damping assembly includes the viscous grease damper; and the viscous grease damper includes an annular viscous grease damper positioned between the base plate and the end surface of the hollow columnar body, and/or the viscous grease damper includes a hollow columnar viscous grease damper positioned between the outer surface of the rotary shaft and the inner surface of the hollow columnar body.

In an embodiment, the rotation compensation mechanism further includes: a first sealing member and a second sealing member, clamped between the active rotation member and the driven rotation member, where

the viscous grease damper includes the annular viscous grease damper positioned between the base plate and the end surface of the hollow columnar body, and the first sealing member and the second sealing member are respectively positioned on the outer side and the inner side of the annular viscous grease damper; and/or
the viscous grease damper includes the hollow columnar viscous grease damper positioned between the outer surface of the rotary shaft and the inner surface of the hollow columnar body, and the first sealing member and the second sealing member are respectively positioned at two opposite ends of the hollow columnar viscous grease damper.

In an embodiment, the damping assembly includes a magnetic damping assembly; and the magnetic damping assembly includes: a first magnetic member and a second magnetic member that are oppositely positioned, where one of the first magnetic member and the second magnetic member is positioned on the side of the driven rotation member facing the active rotation member, the other is positioned on the side of the active rotation member facing the driven rotation member, and the first magnetic member and the second magnetic member are attracted to each other by magnetism to generate torque between the first magnetic member and the second magnetic member.

To resolve the technical problem, another technical solution applied to this application is to provide a medicine delivery dispenser, comprising: a rotation compensation mechanism, which is the rotation compensation mechanism resolved in the foregoing; and
a driving assembly, connected to an active rotation member, and configured to drive the active rotation member to rotate.

The embodiments of this application provide a rotation compensation mechanism and a medicine delivery dispenser. In the rotation compensation mechanism, an active rotation member is positioned and configured to be connected to a driving assembly and rotate under an action of the driving assembly; a driven rotation member is positioned, rotatably connected to the active rotation member and configured to wind a to-be-wound object; and a damping assembly is positioned between the active rotation member and the driven rotation member, to enable the active rotation member to apply driving torque to the driven rotation member through the damping assembly, where the driving torque is not greater than a preset threshold, and the preset threshold is driving torque when the active rotation member rotates relatively to the driven rotation member. In this way, when the driving torque is less than the preset threshold, the driven rotation member can rotate with the active rotation member, to wind the to-be-wound object around the driven rotation member. When the driving torque is equal to the preset threshold, the driven rotation member can rotate relative to the active rotation member, to perform a stroke compensation function. In addition, the active rotation member can rotate clockwise or counterclockwise, which can satisfy requirements of different rotation directions, and does not need a fool-proof design.

### BRIEF DESCRIPTION OF THE DRAWINGS

To describe the technical solutions of the embodiments of this application more clearly, the following briefly introduces accompanying drawings describing the embodiments. The accompanying drawings in the following descriptions show only some embodiments of this application, and a person of ordinary skill in the art can still derive other embodiments from these accompanying drawings.
FIG. 1 is a perspective view of an overall structure of a rotation compensation mechanism according to an embodiment of this application;
FIG. 2 is an exploded view of the structure shown in FIG. 1;
FIG. 3 is a cross-sectional view of a rotation compensation mechanism corresponding to FIG. 2;
FIG. 4 is a structural diagram of a damping assembly and a base plate according to an embodiment of this application;
FIG. 5 is a structural diagram of a damping assembly and a base plate according to an embodiment of this application;
FIG. 6 is a structural diagram of a damping assembly and a base plate according to an embodiment of this application;
FIG. 7 is an exploded, cross-sectional side view of an active rotation member and a driven rotation member;
FIG. 8 is a cross-sectional side view of an elastic material damper positioned between the outer surface of a rotary shaft and the inner surface of a hollow columnar body;
FIG. 9 is an exploded view of the structure shown in FIG. 1;
FIG. 10 is a cross-sectional side view of a rotation compensation mechanism corresponding to FIG. 9;
FIG. 11 is a cross-sectional side view of the structure shown in FIG. 1;
FIG. 12 is a top view of a viscous grease damper, a first sealing member, and a second sealing member on a base plate in FIG. 11;
FIG. 13 is an exploded view of the structure shown in FIG. 1;
FIG. 14 is a cross-sectional side view of a rotation compensation mechanism corresponding to FIG. 13; and
FIG. 15 is a perspective diagram of an internal structure of a medicine delivery dispenser according to an embodiment of this application.

Descriptions of reference numerals:
10. rotation compensation mechanism; 1. active rotation member; 11. base plate; 12. rotary shaft; 121. upper end portion; 2. driven rotation member; 20. hollow columnar body; 21. hole; 22. hook portion; 23. inner surface; 24. outer surface; 25. flange; 31. elastic material damper; 311. first elastic material damper; 312. second elastic material damper; 313. third elastic material damper; 314. fourth elastic material damper; 32. viscous grease damper; 33. magnetic damping assembly; 331. first magnetic member; 332. second magnetic member; A. first engaging groove; B. second engaging groove; a. first sealing member; and b. second sealing member.

### DETAILED DESCRIPTION

Embodiments of this application are clearly and completely described in the following with reference to the accompanying drawings. The described embodiments are merely some rather than all of the embodiments of this application. Based on the embodiments of this application, all other embodiments obtained by a person of ordinary skill in the art without creative efforts shall fall within the protection scope of this application.

The terms "first", "second", and "third" in this application are merely intended for a purpose of description, and shall not be understood as indicating or implying relative significance or implicitly indicating the number of indicated technical features. Therefore, features defined by "first", "second", and "third" can explicitly or implicitly include at least one of the features. In the descriptions of this application, "a plurality of" means at least two, such as two or three unless it is specifically defined otherwise. All directional indications (for example, upper, lower, left, right, front, and rear) in the embodiments of this application are merely used for explaining relative position relationships, movement situations, or the like between various components in a specific posture (as shown in the accompanying drawings). If the specific posture changes, the directional indications change accordingly. In addition, the terms "include", "have", and any variants thereof are intended to cover non-exclusive inclusion. For example, a process, method, system, product, or device that includes a series of steps or units is not limited to the listed steps or units; and instead, further optionally includes a step or unit that is not listed, or further optionally includes another step or unit that is intrinsic to the process, method, product, or device. Thus, use of the word "include" or "have" is interchangeable with "comprise" unless stated otherwise.

"Embodiment" mentioned in the specification means particular features, structures, or characteristics described with reference to the embodiment can be included in at least one embodiment of this application. The term appearing at different sections of this specification can refer to the same embodiment or an independent or alternative embodiment that is mutually exclusive with another embodiment. It is explicitly and implicitly understood by a person skilled in the art that the embodiments described herein can be combined with other embodiments.

In this application, a stroke compensation effect is achieved by using a rotation compensation mechanism 10 that can generate torque. The torque can be generated by using a friction force generated after an elastic material is compressed, viscosity of viscous grease, a magnetic attraction force of a magnetic material, or the like. For a powder-delivery inhaler device with two medicine strips, the rotation compensation mechanism 10 of this application can be universal in some embodiments.

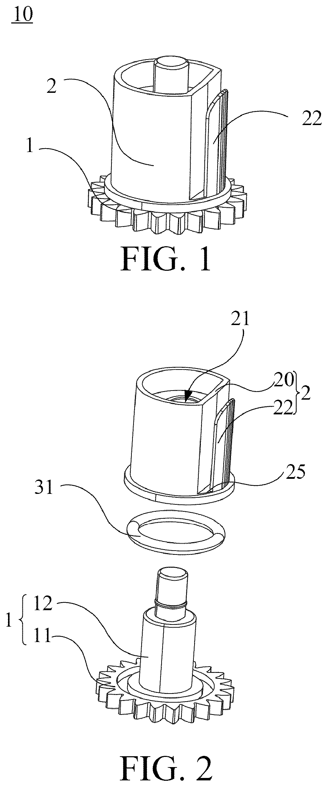

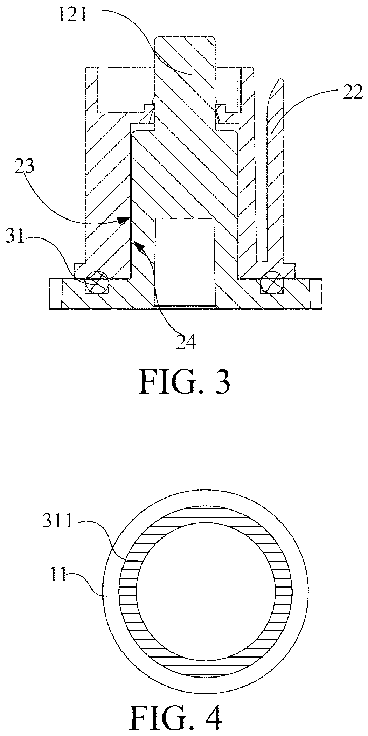

FIG. 1 is a perspective view of an overall structure of a rotation compensation mechanism according to an embodiment of this application; FIG. 2 is an exploded view of the structure shown in FIG. 1; and FIG. 3 is a cross-sectional side view of a rotation compensation mechanism corresponding to FIG. 2. In this embodiment, rotation compensation mechanism 10 is provided. The rotation compensation mechanism 10 can be universal in a powder-delivery inhaler device with two medicine strips. The rotation compensation mechanism 10 includes an active rotation member 1, a driven rotation member 2, and a damping assembly 31.

The active rotation member 1 is configured to be connected to a driving assembly and rotate under a driving action of the driving assembly. The driven rotation member 2 is rotatably connected to the active rotation member 1 and configured to wind a to-be-wound object. The active rotation member 1 can be a rotation gear. The to-be-wound object can be a cover strip, a blister strip, or a base strip, involved in the following embodiments.

Specifically, the active rotation member 1 can rotate clockwise or rotate counterclockwise under driving by the driving assembly, and the active rotation member 1 can apply the same driving torque to the driven rotation member 2 during clockwise rotation and counterclockwise rotation to ensure that a force of the active rotation member 1 driving the driven rotation member 2 to rotate clockwise or rotate counterclockwise is the same. In this way, the rotation compensation mechanism 10 can be used at a position required for clockwise rotation or can be used at a position required for counterclockwise rotation. For example, in a powder-delivery inhaler device with two medicine strips, rotation direction requirements of stroke compensation mechanisms corresponding to the two medicine strips are opposite. The rotation compensation mechanism 10 provided in this application can be universal in the powder-delivery inhaler device, and a rotation compensation mechanism rotating in an opposite direction does not need to be provided. The rotation compensation mechanism 10 has relatively strong universality.

Specifically, the medicine strip mentioned above is a medicine carrier, and is usually an elongated peelable medicine strip. A person skilled in the art can learn that the medicine strip has a plurality of pouches used for containing a medicine, where the pouches are uniformly spaced apart in the length direction and are defined between two peelable pieces fastened to each other. After being peeled, the medicine strip is divided into two parts: a cover strip and a blister. The cover strip is the to-be-wound object mentioned in all the embodiments of this application. In some embodiments, the to-be-wound object in this application can alternatively be a blister sheet, a base strip, or a cover strip that are described herein. The medicine can be in the form of a capsule, a pill, a tablet, or a powder. In some embodiments, the medicine includes a medicine in the form of a powder.

In some embodiments, the rotation compensation mechanism 10 comprises at least one of the active rotation member 1 with a rotary shaft 12, and the driven rotation member 2 with a hole 21. An upper end portion 121 of the rotary shaft 12 is inserted into the hole 21 to connect the active rotation member 1 and the driven rotation member 2. Specifically, the active rotation member 1 can include rotary shaft 12 and a base plate 11. The base plate 11 is plate- or disc-shaped. An outer peripheral edge of the base plate 11 has serrations to be engaged with a driving assembly, so that by using a driving member in the driving assembly, the base plate 11 can be driven to rotate. The driving member can be a motor, a pump, or manually activated. The rotary shaft 12 is vertically connected to a side surface of the base plate 11; the rotary shaft 12 and the base plate 11 form an integral active rotation member 1; and the rotary shaft 12 extends from the base plate 11.

Referring to FIG. 1, FIG. 2, and FIG. 3, in this embodiment, the active rotation member 1 has the rotary shaft 12, and the driven rotation member 2 includes a hollow columnar body 20 having the hole 21. The driven rotation member 2 is sleeved on the rotary shaft 12 through the hole 21, to implement a connection between the active rotation member 1 and the driven rotation member 2. Specifically, the upper end portion 121 of the rotary shaft 12 passes through the hole 21 at an end surface of the driven rotation member 2 and protrudes from the driven rotation member 2. As shown in FIG. 3, the driven rotation member 2 includes the hollow columnar body 20 and a hook portion 22 located on an outer side of a side wall of the hollow columnar body 20. Specifically, the hollow columnar body 20 is cylindrical, and a part of the side wall is planar. A flange 25 is provided on the end portion of the hollow columnar body 20. In some embodiments, the hook portion 22 is fin or blade-shaped. The hook portion 22 is connected to the flange 25 positioned at an end portion of the hollow columnar body 20. The hook portion 22 extends from the flange 25 and is parallel to and spaced from the planar side wall of the hollow columnar body 20. The hook portion 22 is configured to receive a to-be-wound object, and one end of the to-be-wound object is fixed, fastened, or otherwise connected thereto. When the rotation compensation mechanism 10 rotates, the to-be-wound object is enabled to wrap around an outer peripheral surface of the side wall of the driven rotation member 2.

Damping assembly 31 is positioned between the active rotation member 1 and the driven rotation member 2, and is configured to generate torque between the active rotation member 1 and the driven rotation member 2. This enables the active rotation member 1 to apply driving torque to the driven rotation member 2 through the damping assembly. In other words, the damping assembly 31 is configured to generate a rotational friction force between the active rotation member 1 and the driven rotation member 2 to enable the active rotation member 1 during rotation to drive the driven rotation member 2 to rotate together. Specifically, the damping assembly 31 can be positioned between the base plate 11 and the end surface of the hollow columnar body 20 to enable the active rotation member 1 to provide the driving torque from between the base plate 11 and the end surface of the hollow columnar body 20 for the driven rotation member 2 through the damping assembly 31. In some embodiments, the damping assembly 31 can alternatively be positioned between the outer surface 24 of the rotary shaft 12 and the inner surface 23 of the hollow columnar body 20; or the damping assembly can be positioned between the base plate 11 and the end surface of the hollow columnar body 20 and between the outer surface 24 of the rotary shaft 12 and the inner surface 23 of the hollow columnar body 20. In this way, the active rotation member 1 provides, from between the base plate 11 and the end surface of the hollow columnar body 20, and/or between the outer surface 24 of the rotary shaft 12 and the inner surface 23 of the hollow columnar body 20, the driving torque to the driven rotation member 2 through the damping assembly to enable the active rotation member 1 to drive the driven rotation member 2 to rotate. It should be noted that each end surface of the hollow columnar body 20 involved in this application refers to the side surface of the driven rotation member 2 facing the base plate 11.

The driving torque is limited to a preset threshold. The preset threshold is driving torque when the active rotation member 1 rotates relative to the driven rotation member 2. In this way, when the driving torque is less than the preset threshold, the driven rotation member 2 can rotate with the active rotation member 1, to wind the to-be-wound object around the driven rotation member 2. When the driving torque is equal to the preset threshold, the driven rotation member 2 can rotate relative to the active rotation member 1, to perform a stroke compensation function. In addition, the driven rotation member 2 can alternatively be driven to rotate by driving torque of the preset threshold, and implement winding of the to-be-wound object. In addition, the active rotation member 1 can rotate clockwise or counterclockwise, which can satisfy requirements of different rotation directions.

A person skilled in the art can understand that the to-be-wound object is wound around the driven rotation member 2. The to-be-wound object is wound by rotation of the driven rotation member 2 driven by the active rotation member 1. During winding of the to-be-wound object, the to-be-wound object provides the driven rotation member 2 with an action force with a direction opposite to the rotation direction of the driven rotation member 2, to prevent the driven rotation member 2 from rotating with the active rotation member 1. In other words, the rotation of the driven rotation member needs driving torque of a specific magnitude, and the magnitude of the driving torque depends on a magnitude of the action force applied by the to-be-wound object to the driven rotation member. In practice, generally, the action force applied by the wound object to the driven rotation member needs to be less than a limit value, to prevent the to-be-wound object from being damaged by an excessively large action force. In some implementations, a rotation of a single winding of the to-be-wound object needs to be consistent to ensure that in each medicine delivery process the medicine is accurately and uniformly distributed to a preset position for being inhaled by a patient. Therefore, because after some of the to-be-wound object is wound around the driven rotation member 2, the radius of the driven rotation member 2 increases, resulting in an increase in the circumference of the driven rotation member 2. The means one full rotation of a single winding will wind a longer length of the to-be-wound object than when the circumference was smaller. As a result, the action force applied to the to-be-wound object gradually increases for each full rotation as increasing amounts of the to-be-wound object are wrapped around the driven rotation member 2, and ultimately the driving torque also increases.

After the driving torque increases to a preset threshold, the driven rotation member 2 rotates relative to the active rotation member 1, and rotation angles of the driven rotation member 2 and the active rotation member 1 are inconsistent. Stated differently, the friction value of the damping assembly is overcome when the driving torque increases or exceeds the preset threshold, so while the active rotation member 1 rotates the same amount, the driven rotation member 2 "slips" and rotates an amount less than that of the active rotation member 1. In a specific implementation, the rotation angle of the driven rotation member 2 is slightly less than the rotation angle of the active rotation member 1. Therefore, a rotation of the to-be-wound object driven by the driven rotation member 2 also tends to be consistent with a previous rotation; and an increased rotation, that is, a rotation compensation effect, is not generated due to the increase of the radius of the driven rotation member 2. In practice, a value of the preset threshold is directly related to a type and parameter setting of the damping assembly, and this value can be preset according to a design requirement. This can be understood as the to-be-wound object applies specific torque to the driven rotation member 2; when the torque is less than the preset threshold, the driven rotation member 2 rotates with the active rotation member 1; and when the torque is equal to or exceeds the preset threshold, the driven rotation member 2 rotates at a different rate relative to the active rotation member 1 to achieve the rotation compensation effect.

In some embodiments, the damping assembly includes one or more of an elastic material damper 31, a viscous grease damper 32, and a magnetic damping assembly 33. In some instances, the damping assembly does not include an elastic damper made of a rigid material.

In an embodiment, as shown in FIG. 3, the damping assembly includes an elastic material damper 31. The elastic material damper 31 is located between the driven rotation member 2 and the active rotation member 1. After the driven rotation member 2 and the active rotation member 1 are mounted, the elastic material damper 31 is in a specific compressed state, and a specific elastic force is generated. Further, a specific friction force is generated between the driven rotation member 2 and the elastic material damper 31, and between the elastic material damper 31 and the active rotation member 1, to generate corresponding torque. The elastic material can be a flexible material such as rubber or silicone.

In a specific embodiment, with reference to FIG. 3, the elastic material damper 31 is positioned between the base plate 11 and the end surface of the hollow columnar body 20.

FIG. 4 shows a damping assembly and a base plate 11 according to an embodiment of this application. The damping assembly includes a first elastic material damper 311 positioned between the base plate 11 and the end surface of the hollow columnar body 20. The first elastic material damper 311 is in the shape of a closed loop, for example, in the shape of a closed circular ring. In this way, the first elastic material damper 311 can be used to perform a sealing function, to prevent external dirt particles or a powder of the to-be-wound object from entering an inner ring of the rotation compensation mechanism 10, thereby preventing the damping assembly in the inner ring from being affected.

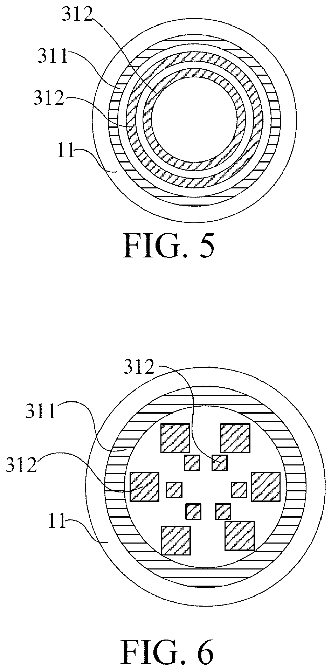

In FIG. 5, a damping assembly and a base plate according to another embodiment of this application are shown. The damping assembly further includes one or more second elastic material dampers 312. The plurality of second elastic material dampers 312 are positioned at intervals. Each second elastic material damper 312 is positioned between the base plate 11 and the end surface of the hollow columnar body 20 and is located on the side of the first elastic material damper 311 close to the rotary shaft 12. The first elastic material damper 311 is specifically positioned in a circle around the periphery of the one or more second elastic material dampers 312.

In an embodiment, as shown in FIG. 5, each second elastic material damper 312 is in the shape of a closed loop. In this way, the second elastic material damper 312 in the shape of the closed loop can be further used to perform a sealing function, and the damping assembly generates uniform torque between the active rotation member 1 and the driven rotation member 2 in the circumferential direction of the rotary shaft 12, to facilitate stable rotation of the rotation compensation mechanism 10.

In some instances, the first elastic material damper 311 and the plurality of second elastic material dampers 312 are coaxially sleeved. Specifically, the first elastic material damper 311 and each second elastic material damper 312 can be both sleeved around the rotary shaft 12.

In another specific embodiment, FIG. 6 is a schematic structural diagram of a damping assembly and a base plate according to another embodiment of this application. Each second elastic material damper 312 is in the sheet shape, and a plurality of second elastic material dampers 312 in the sheet shape are positioned at intervals in the circumferential direction of the rotary shaft 12 and form at least one ring shape. The sheet shape can be any shape, such as a rectangle, a circle, an arc, or a sector. Specifically, the first elastic material damper 311 and the at least one ring shape formed by the plurality of second elastic material dampers 312 in the sheet shape are coaxially nested, and can both extend around the rotary shaft 12.

In an embodiment, a person skilled in the art can understand that when a plurality of second elastic material dampers 312 are provided, the plurality of second elastic material dampers 312 can alternatively be partially in the shape of a closed loop and partially in the sheet shape. In addition, the first elastic material damper 311 can alternatively be in the sheet shape. Based on this, a sealing element, such as a sealing ring, can be positioned, to seal the rotation compensation mechanism 10.

Since the first elastic material damper 311 is located in an outer ring, the first elastic material damper 311 is easily affected and damaged by external dirt particles, a powder of the to-be-wound object, or the like. To reduce the impact caused by the friction force generated between the whole damping assembly and the active rotation member 1 and the driven rotation member 2 due to damage of the first elastic material damper 311, torque generated between the active rotation member 1 and the driven rotation member 2 by the first elastic material damper 311 can be far less than torque generated between the active rotation member 1 and the driven rotation member 2 by each second elastic material damper 312. For example, the torque generated between the active rotation member 1 and the driven rotation member 2 by the first elastic material damper 311 is less than the torque generated between the active rotation member 1 and the driven rotation member 2 by each second elastic material damper 312 by more than an order of magnitude. In this way, even if the first elastic material damper 311 is damaged, a proportion of a change of the torque generated between the active rotation member 1 and the driven rotation member 2 by the whole damping assembly is not excessively large, so that service life of the rotation compensation mechanism 10 can be effectively prolonged.

In some embodiments, a cross-sectional area of the first elastic material damper 311 and a cross-sectional area of the second elastic material damper 312 are the same, and the first elastic material damper 311 and the second elastic material damper 312 are both in the shape of the closed loop, that is, the first elastic material damper 311 and the second elastic material damper 312 have a substantially same compression deformation amount. In addition, the elastic coefficient and/or friction coefficient of the first elastic material damper 311 is smaller than the elastic coefficient and/or the friction coefficient of each second elastic material damper 312, so that the torque generated between the active rotation member 1 and the driven rotation member 2 by the first elastic material damper 311 is less than the torque generated between the active rotation member 1 and the driven rotation member 2 by each second elastic material damper 312. It should be noted that referring to FIG. 3, the cross-sectional area of the elastic material damper 31 involved in this application is the cross-sectional area of the elastic material damper 31 in the vertical direction of the rotation compensation mechanism 10.

A difference between the elastic coefficient and/or the friction coefficient of the first elastic material damper 311 and the elastic coefficient and/or the friction coefficient of the second elastic material damper 312 can be set according to the specific application, so long as the torque generated between the active rotation member 1 and the driven rotation member 2 by each second elastic material damper 312 is greater than the torque generated between the active rotation member 1 and the driven rotation member 2 by the first elastic material damper 311.

In some embodiments, the elastic coefficient of the first elastic material damper 311 can be the same as the elastic coefficient of each second elastic material damper 312, and the cross-sectional area of the first elastic material damper 311 can be less than the cross-sectional area of each second elastic material damper 312. Alternatively, the elastic coefficient and the cross-sectional area of each second elastic material damper 312 are both greater than the elastic coefficient and the cross-sectional area of the first elastic material damper 311. It can be understood that in the foregoing implementation, a significant difference between the torque generated by the first elastic material damper 311 and the second elastic material damper 312 can be implemented by adjusting parameters such as an elastic system, the friction coefficient, and a compression deformation amount of the first elastic material damper 311 and the second elastic material damper 312. This permits the avoidance of significant impact on the torque of the whole device in the event that one of the first elastic material damper 311 and the second elastic material damper 312 is abnormally affected or damaged.

For example, when a plurality of second elastic material dampers 312 is provided, torque generated between the active rotation member 1 and the driven rotation member 2 by the plurality of second elastic material dampers 312 gradually increases in the direction closer to the rotary shaft 12. In this way, after the elastic material damper 31 in the outer ring is damaged and fails, a proportion of an impact of the elastic material damper 31 on the torque generated between the active rotation member 1 and the driven rotation member 2 by the whole damping assembly is reduced, thereby effectively performing normal operation of the rotation compensation mechanism 10.

A person skilled in the art can understand that, when a plurality of second elastic material dampers 312 are all in the shape of the closed loop and are coaxially positioned around the rotary shaft 12, the torque generated between the active rotation member 1 and the driven rotation member 2 by the plurality of second elastic material dampers 312 gradually increases in the direction closer to the rotary shaft 12. When the plurality of second elastic material dampers 312 are all in a sheet shape and wound to form a plurality of ring shapes, the torque generated between the active rotation member 1 and the driven rotation member 2 by the second elastic material dampers 312 corresponding to the plurality of ring shapes gradually increases in the direction closer to the rotary shaft 12. For example, the plurality of second elastic material dampers 312 in the sheet shape form three ring shapes, a first ring shape is the closest to the first elastic material damper 311, a second ring shape is the second closest, and a third ring shape is the farthest. In this case, torque generated between the active rotation member 1 and the driven rotation member 2 by a plurality of second elastic material dampers 312 corresponding to the first ring shape is less than torque generated between the active rotation member 1 and the driven rotation member 2 by a plurality of second elastic material dampers 312 corresponding to the second ring shape, and the torque generated between the active rotation member 1 and the driven rotation member 2 by the plurality of second elastic material dampers 312 corresponding to the second ring shape is less than torque generated between the active rotation member 1 and the driven rotation member 2 by a plurality of second elastic material dampers 312 corresponding to the third ring shape.

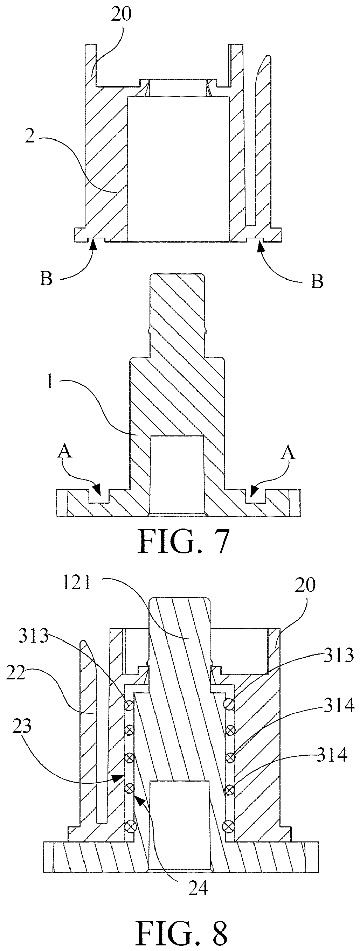

In an embodiment, FIG. 7 shows an exploded cross-sectional view of an active rotation member 1 and a driven rotation member 2. A first engaging groove A is provided on the side surface of the base plate 11 facing the end surface of the hollow columnar body 20, and the first elastic material damper 311 or the second elastic material damper 312 is partially, elastically compressed in the first engaging groove A and abuts against the bottom wall of the first engaging groove A. The end surface of the hollow columnar body 20 is a flat surface, and is attached to the surface of the active rotation member 1 to compress the first elastic material damper 311 or the second elastic material damper 312 located in the first engaging groove A. Torque is generated between the driven rotation member 2 and the elastic material damper 31 and between the elastic material damper 31 and the active rotation member 1 by using the first elastic material damper 311 and/or the second elastic material damper 312. Specifically, one first engaging groove A corresponds to one first elastic material damper 311 or one second elastic material damper 312. A quantity of first engaging grooves A is the same as a sum of a quantity of the first elastic material dampers 311 and a quantity of second elastic material dampers 312.

Again referring to FIG. 7, a second engaging groove B can alternatively be provided in some embodiments on the end surface of the hollow columnar body 20. The first elastic material damper 311 or the second elastic material damper 312 is partially elastically compressed in the second engaging groove B and abuts against the bottom wall of the second engaging groove B. The surface of the base plate 11 at a position corresponding to the end surface of the hollow columnar body 20 is a flat surface, and is attached to the end surface of the hollow columnar body 20 to compress the first elastic material damper 311 or the second elastic material damper 312 located in the second engaging groove B. Specifically, one second engaging groove B corresponds to one first elastic material damper 311 or one second elastic material damper 312. A quantity of second engaging grooves B is the same as a sum of a quantity of first elastic material dampers 311 and a quantity of second elastic material dampers 312.

In some instances, a first engaging groove A can also be provided on the side surface of the base plate 11 facing the end surface of the hollow columnar body 20, and the first elastic material damper 311 or the second elastic material damper 312 can be partially elastically compressed in the first engaging groove A and abut against the bottom wall of the first engaging groove A. Additionally, a second engaging groove B is provided on the end surface of the hollow columnar body 20, and the first elastic material damper 311 or the second elastic material damper 312 is partially elastically compressed in the second engaging groove B and abuts against the bottom wall of the second engaging groove B. Ad sum of the groove depths of the first engaging groove A and the second engaging groove B is less than the thickness of the first elastic material damper 311 or less than the thickness of the second elastic material damper 312. One first engaging groove A is provided corresponding to one second engaging groove B. With reference to FIG. 3, the thickness refers to the dimension of the elastic material damper 31 in the vertical direction.

By providing the first engaging groove A and/or the second engaging groove B, the first elastic material damper 311 and/or the second elastic material damper 312 can be limited to prevent relative displacement from occurring among the first elastic material damper 311 and/or the second elastic material damper 312, the base plate 11, and the driven rotation member 2 during operation of the rotation compensation mechanism 10, thereby preventing any interference with the rotation compensation effect.

In another embodiment, FIG. 8 shows an elastic material damper positioned between the outer surface of a rotary shaft and the inner surface of a hollow columnar body. The first elastic material damper 311 and/or the second elastic material damper 312 can alternatively be positioned between the outer surface 24 of the rotary shaft 12 and the inner surface 23 of the hollow columnar body 20 in some instances. In some cases, the first elastic material damper 311 and/or the second elastic material damper 312 can be positioned between the base plate 11 and the end surface of the hollow columnar body 20, and between the outer surface 24 of the rotary shaft 12 and the inner surface 23 of the hollow columnar body 20. For ease of distinguishing, the first elastic material damper 311 positioned between the outer surface 24 of the rotary shaft 12 and the inner surface 23 of the hollow columnar body 20 is referred to as a third elastic material damper 313 below; and the second elastic material damper 312 positioned between the outer surface 24 of the rotary shaft 12 and the inner surface 23 of the hollow columnar body 20 is referred to as a fourth elastic material damper 314.

As shown in FIG. 8, the damping assembly includes a third elastic material damper 313. The third elastic material damper 313 is positioned between the outer surface 24 of the rotary shaft 12 and the inner surface 23 of the hollow columnar body 20; and the third elastic material damper 313 is in the shape of a closed loop, for example, in the shape of a closed circular ring. In this way, the third elastic material damper 313 can be used to perform a sealing function, to prevent external dirt particles or powder of the to-be-wound object from entering the inner ring of the rotation compensation mechanism 10 to affect a part of the damping assembly in the inner ring.

In an embodiment, referring to FIG. 8, the damping assembly can further include one or more fourth elastic material dampers 314. A plurality of fourth elastic material dampers 314 can be positioned at intervals between the outer surface 24 of the rotary shaft 12 and the inner surface 23 of the hollow columnar body 20 in the axial direction of the rotary shaft 12. Each fourth elastic material damper 314 can be located on the side of the third elastic material damper 313 close to the base plate 11.

In an embodiment, each fourth elastic material damper 314 is in the shape of a closed loop. In this way, the fourth elastic material damper 314 in the shape of the closed loop can be further used to perform a sealing function. The damping assembly generates uniform torque between the active rotation member 1 and the driven rotation member 2 in the circumferential direction of the rotary shaft 12 to facilitate stable rotation of the rotation compensation mechanism 10.

Each elastic material damper 31 is in the shape of the closed circular ring, where the third elastic material damper 313 closest to the side of the hole 21 of the driven rotation member 2 can perform a sealing function to prevent powder of the medicine from further entering the interior of the rotation compensation mechanism 10 and prevent the plurality of fourth elastic dampers from losing an effect. In addition, the third elastic material damper 313 and the fourth elastic material damper 314 can both enable the active rotation member 1 to apply the driving torque to the driven rotation member 2.

In another embodiment, each fourth elastic material damper 314 is in a sheet shape, and a plurality of fourth elastic material dampers 314 are positioned at intervals in the circumferential direction of the rotary shaft 12 and form at least one ring shape. A specific distribution manner of the fourth elastic material dampers 314 in the sheet shape on the rotary shaft 12 is similar to the specific distribution manner in which the plurality of second elastic materials dampers in the sheet shape are positioned at intervals on the base plate 11 and form at least one ring shape. For details, refer to the foregoing descriptions, and details are not described herein again. In addition, similar to the distribution manner of the first elastic material damper 311 and the second elastic material damper 312, some of the fourth elastic material dampers 314 can alternatively be in the shape of a closed loop and some of the fourth elastic material dampers 314 can be in the sheet shape, and the third elastic material damper 313 can alternatively be in the sheet shape. The distribution manner of the third elastic material damper 313 and the distribution manner of the fourth elastic material damper 314 can be randomly combined.

Similar to the foregoing, it can be understood that the third elastic material damper 313 is also easy to be damaged by external dirt particles, powder of the to-be-wound object, or the like. Therefore, torque generated between the active rotation member 1 and the driven rotation member 2 by the third elastic material damper 313 can be far less than torque generated between the active rotation member 1 and the driven rotation member 2 by each fourth elastic material damper 314. In this way, even if the third elastic material damper 313 is damaged, a proportion of a change of the torque generated between the active rotation member 1 and the driven rotation member 2 by the whole damping assembly is not excessively large, so that a service life of the rotation compensation mechanism 10 can be effectively prolonged. A specific implementation is similar to the implementation in which the torque generated between the active rotation member 1 and the driven rotation member 2 by the first elastic material damper 311 can be far less than the torque generated between the active rotation member 1 and the driven rotation member 2 by each second elastic material damper 312.

In some embodiments, the third elastic material damper 313 and the fourth elastic material damper 314 have the same cross-sectional area and are both in the shape of a closed loop. The elastic coefficient of the third elastic material damper 313 can be less than the elastic coefficient of each fourth elastic material damper 314. The torque generated between the active rotation member 1 and the driven rotation member 2 by the third elastic material damper 313 can be less than the torque generated between the active rotation member 1 and the driven rotation member 2 by each fourth elastic material damper 314. Similar to the first elastic material damper 311 and the second elastic material damper 312, the elastic coefficient and/or the cross-sectional area of the third elastic material damper 313 and the fourth elastic material damper 314 can be adjusted. Thus, torque generated between the active rotation member 1 and the driven rotation member 2 by the third elastic material damper 313 can be less than the torque generated between the active rotation member 1 and the driven rotation member 2 by each fourth elastic material damper 314. Specific size relationships of the coefficient and/or the cross-sectional area are similar to the foregoing embodiments and details are not described herein again.

In some embodiments, a plurality of fourth elastic material dampers 314 are provided, and torque generated between the active rotation member 1 and the driven rotation member 2 by the plurality of fourth elastic material dampers 314 gradually increases in the direction closer to the base plate 11. The advantage is that, when the third elastic material damper 313 close to the side of the hole 21 of the driven rotation member 2 is affected by medicine powder or the like, a change of the overall friction force generated between the active rotation member 1 and the driven rotation member 2 by the damping assembly is not excessively large.

A person skilled in the art can understand that, when the plurality of fourth elastic material dampers 314 are all in the shape of the closed loop and are positioned at intervals in the axial direction of the rotary shaft 12, the torque generated between the active rotation member 1 and the driven rotation member 2 by the plurality of fourth elastic material dampers 314 gradually increases in the direction closer to the base plate 11. When the plurality of fourth elastic material dampers 314 are all in the sheet shape and wound to form a plurality of ring shapes positioned in the axial direction of the rotary shaft 12, torque generated between the active rotation member 1 and the driven rotation member 2 by the fourth elastic material damper 314 corresponding to the plurality of ring shapes gradually increases in the direction closer to the base plate 11. For example, the plurality of fourth elastic material dampers 314 in the sheet shape form three ring shapes, a first ring shape is the closest to the third elastic material damper 313, a second ring shape is the second closest, and a third ring shape is the farthest. In this case, torque generated between the active rotation member 1 and the driven rotation member 2 by a plurality of fourth elastic material dampers 314 corresponding to the first ring shape is less than torque generated between the active rotation member 1 and the driven rotation member 2 by a plurality of fourth elastic material dampers 314 corresponding to the second ring shape. The torque generated between the active rotation member 1 and the driven rotation member 2 by the plurality of fourth elastic material dampers 314 corresponding to the second ring shape is less than torque generated between the active rotation member 1 and the driven rotation member 2 by a plurality of fourth elastic material dampers 314 corresponding to the third ring shape.

In an embodiment, similarly, to prevent the rotation compensation effect from being affected, the third elastic material damper 313 and/or the fourth elastic material damper 314 to prevent relative displacement from occurring between the third elastic material damper 313 and/or the fourth elastic material damper 314 and the rotary shaft 12 and the driven rotation member 2 during operation of the rotation compensation mechanism 10 can be limited. A third engaging groove can be provided on the outer surface 24 of the rotary shaft 12, and/or a fourth engaging groove can be provided on the inner surface 23 of the hollow columnar body 20, and the third elastic material damper 313 or the fourth material damper can be partially elastically compressed in the third engaging groove and abut against the bottom wall of the third engaging groove. The third elastic material damper 313 or the fourth material damper can be elastically compressed in the second engaging groove B and abut against the bottom wall of the second engaging groove B. Finally, the inner surface 23 of the hollow columnar body 20 can be attached to the outer surface 24 of the rotary shaft 12 to compress the third elastic material damper 313 and/or the fourth material damper. One engaging groove corresponds to one elastic material damper 31. When the third engaging groove and the fourth engaging groove are both provided, the third engaging groove and the fourth engaging groove are provided opposite to each other, to jointly hold an elastic material damper 31.

A person skilled in the art can understand that the elastic material damper 31 can be positioned between the base plate 11 and the end surface of the hollow columnar body 20, can be positioned between the outer surface 24 of the rotary shaft 12 and the inner surface 23 of the hollow columnar body 20, or can be positioned both between the base plate 11 and the end surface of the hollow columnar body 20 and between the outer surface 24 of the rotary shaft 12 and the inner surface 23 of the hollow columnar body 20. Specific structures and functions are all described in the foregoing embodiments, and details are not described herein again.

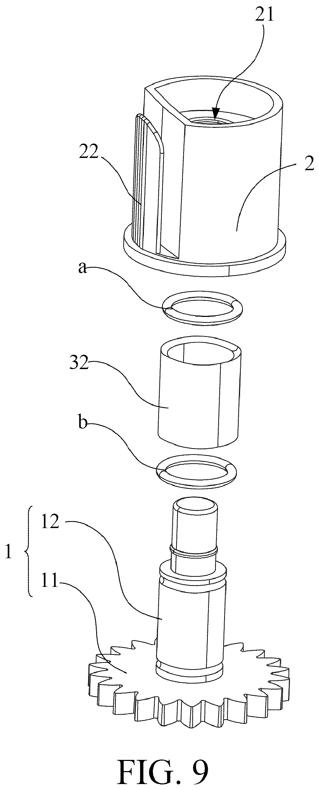

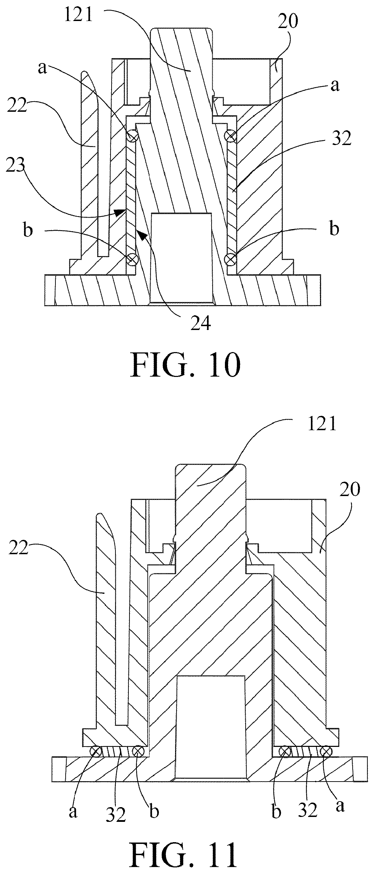

In another embodiment, FIG. 9 shows an exploded view of the structure shown in FIG. 1; FIG. 10 shows a cross-sectional view of a rotation compensation mechanism corresponding to FIG. 9; and the damping assembly is a viscous grease damper 32. The viscous grease damper 32 is located between the driven rotation member 2 and the active rotation member 1, and after the driven rotation member 2 and the active rotation member 1 are mounted, the driven rotation member 2 can rotate relative to the active rotation member 1. The viscous grease damper 32 generates specific torque between the driven rotation member 2 and the active rotation member 1 through stickiness of the viscous grease damper 32. Viscosity of the grease and contact areas between the viscous grease damper 32 and the active rotation member 1 and the driven rotation member 2 determine magnitude of the torque. In some instances, the viscous grease damper 32 can be an existing high-viscosity damping grease formed by an extremely high-viscosity synthetic grease thickened by an inorganic thickener.

In some embodiments, as shown in FIG. 9, the viscous grease damper 32 is of a hollow columnar shape. As shown in FIG. 10, the hollow columnar viscous grease damper 32 can be positioned on the outer surface 24 of the rotary shaft 12, and be located between the outer surface 24 of the rotary shaft 12 and the inner surface 23 of the hollow columnar body 20.

As referenced in FIG. 9 and FIG. 10, the rotation compensation mechanism 10 can further include a first sealing member a and a second sealing member b. The first sealing member a and the second sealing member b are sandwiched between the outer surface 24 of the rotary shaft 12 and the inner surface 23 of the hollow columnar body 20. As shown in FIG. 10, the first sealing member a and the second sealing member b are respectively positioned at the upper end and the lower end that are opposite to each other of the hollow columnar viscous grease damper 32, to avoid a problem in which powder of the to-be-wound object or other external dirt and particles are stuck to the viscous grease damper 32 to affect a damping effect of the viscous grease damper 32 and cause the viscous grease damper 32 to lose an effect. The first sealing member a and/or the second sealing member b can be a sealing ring, for example, a rubber or a silicone ring.

A limiting groove can also be provided at positions corresponding to a first sealing ring and/or a second sealing ring on the outer surface 24 of the rotary shaft 12 and/or the inner surface 23 of the hollow columnar body 20. The first sealing ring and/or the second sealing ring can be located in the limiting groove so that the first sealing ring and/or the second sealing ring are limited by using the limiting groove and a sealing effect is strengthened.

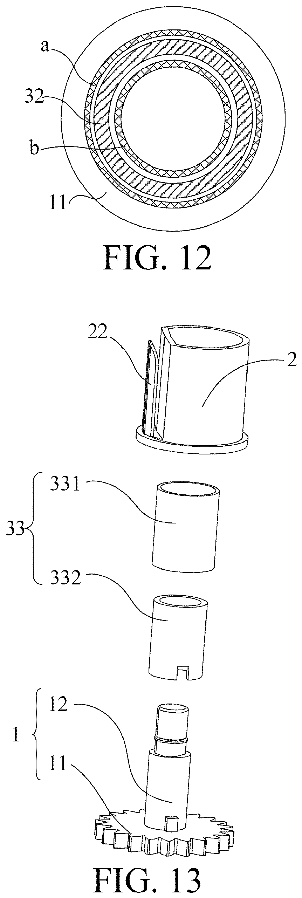

In some embodiments, FIG. 11 shows cross-sectional view of the structure shown in FIG. 1 and FIG. 12 shows a viscous grease damper, a first sealing member, and a second sealing member on a base plate in FIG. 11. The viscous grease damper 32 is annular, and the annular viscous grease damper 32 is positioned between the base plate 11 and the end surface of the hollow columnar body 20. In this embodiment, the first sealing member a and the second sealing member b are specifically positioned between the base plate 11 and the end surface of the hollow columnar body 20, and the first sealing member a and the second sealing member b are respectively positioned on the outer side and the inner side of the annular viscous grease damper 32.

The viscous grease damper 32 can be positioned only between the base plate 11 and the end surface of the hollow columnar body 20; or the viscous grease damper 32 can be positioned only between the outer surface 24 of the rotary shaft 12 and the inner surface 23 of the hollow columnar body 20, which is specifically described above. The viscous grease damper 32 can also be positioned between the base plate 11 and the end surface of the hollow columnar body 20, and between the outer surface 24 of the rotary shaft 12 and the inner surface 23 of the hollow columnar body 20. In this case, one of the first sealing member a and the second sealing member b is positioned at the outer side of the annular viscous grease damper 32, and the other is positioned at the end of the hollow columnar viscous grease damper 32 facing away from the base plate 11. Specific structures and functions are all involved in the foregoing embodiments, and details are not described herein again.

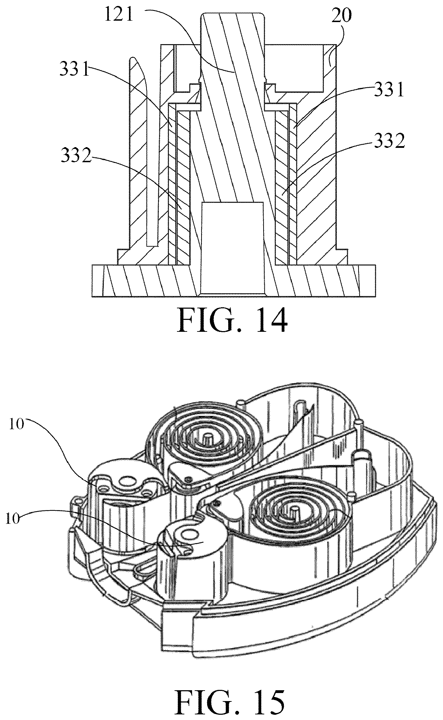

In another embodiment, referring to FIG. 13 and FIG. 14, FIG. 13 shows an exploded view of the structure shown in FIG. 1 and FIG. 14 shows a rotation compensation mechanism corresponding to FIG. 13 where the damping assembly includes a magnetic damping assembly 33. The magnetic damping assembly 33 includes a first magnetic member 331 and a second magnetic member 332 that are positioned oppositely and have opposite magnetism. One of the first magnetic member 331 and the second magnetic member 332 is positioned on the side of the driven rotation member 2 facing the active rotation member 1, and the other is positioned on the side of the active rotation member 1 and facing the driven rotation member 2. After the driven rotation member 2 and the active rotation member 1 are mounted, the first magnetic member 331 and the second magnetic member 332 are attracted to each other by magnetism, to generate torque between the first magnetic member 331 and the second magnetic member 332.

In an embodiment shown in FIG. 13 and FIG. 14, the first magnetic member 331 and the second magnetic member 332 are located between the outer surface 24 of the rotary shaft 12 and the inner surface 23 of the hollow columnar body 20. The first magnetic member 331 can be a ferromagnetic material and be positioned on the side of the inner surface 23 of the hollow columnar body 20 facing the rotary shaft 12. The second magnetic member 332 is a magnet and can be positioned on the side of the rotary shaft 12 facing the inner surface 23 of the hollow columnar body 20. The first magnetic member 331 and the second magnetic member 332 are attracted to each other by magnetism so that torque is generated between the first magnetic member 331 and the second magnetic member 332. Alternatively, the first magnetic member 331 can be a magnet, and the second magnetic member 332 can be a ferromagnetic material.

In another embodiment, the magnetic damping assembly 33 comprises the first magnetic member 331 and the second magnetic member 332 positioned between the base plate 11 and the end surface of the hollow columnar body 20. The first magnetic member 331 can be a ferromagnetic material and be positioned on the side of the end surface of the hollow columnar body 20 facing the base plate 11. The second magnetic member 332 can be a magnet and be positioned on the side of the end surface of the base plate 11 facing the hollow columnar body 20. The first magnetic member 331 and the second magnetic member 332 are attracted to each other by magnetism to generate torque between the first magnetic member 331 and the second magnetic member 332. Alternatively, the first magnetic member 331 can be a magnet, and the second magnetic member 332 can be a ferromagnetic material.

The magnetic damping assembly 33 can be positioned only between the outer surface 24 of the rotary shaft 12 and the inner surface 23 of the hollow columnar body 20; or can be positioned only between the base plate 11 and the end surface of the hollow columnar body 20. The magnetic damping assembly 33 can also be positioned between the base plate 11 and the end surface of the hollow columnar body 20, and between the outer surface 24 of the rotary shaft 12 and the inner surface 23 of the hollow columnar body 20. Specific structures and functions are all involved in the foregoing embodiments, and details are not described herein again.

The rotation compensation mechanism 10 described herein comprises an active rotation member 1, a driven rotation member 2, and a damping assembly. The active rotation member 1 is configured to be connected to a driving assembly and rotate under an action of the driving assembly. The driven rotation member 2 connected to the active rotation member 1 is configured to wind a to-be-wound object. A damping assembly is positioned between the active rotation member 1 and the driven rotation member 2, so that torque between the active rotation member 1 and the driven rotation member 2 is generated through the damping assembly to enable the active rotation member 1 to apply driving torque to the driven rotation member 2 through the damping assembly. When the driving torque is less than a preset threshold, the driven rotation member 2 can rotate with the active rotation member 1, to wind the to-be-wound object around the driven rotation member 2. When the driving torque is equal to the preset threshold, the driven rotation member 2 can rotate relative to the active rotation member 1. Torque is generated by using a friction force generated by compression of an elastic material, viscous grease, and a magnetic material. The rotation compensation mechanism 10 can perform a rotation compensation function, and the active rotation member 1 can rotate clockwise or counterclockwise to meet requirements of different rotation directions. The rotation compensation mechanism can be universal in a powder-delivery inhaler device with two medicine strips. In addition, compared with conventional approaches in which a rigid elastic material is used as a rotation compensation mechanism, in the solution of the embodiments of this application, after each single compensation process ends, the compensation mechanism no longer generates a continuously large pulling force for the medicine strip. The result is that a pulling force impact on a mechanism of other inhaler features, such as the medicine strip, can be reduced, and design complexity of those mechanisms can be reduced. For example, a need for a ratchet mechanism can be reduced.

FIG. 15 is a perspective view of a diagram of an internal structure of a medicine delivery dispenser according to an embodiment of this application. In this embodiment, a medicine delivery dispenser is provided. The medicine delivery dispenser usually includes a main body or a housing, and a medicine carrier located in the main body or the housing of the medicine delivery dispenser. The medicine carrier can be in the form of an elongated blister strip, and the blister strip comprises a plurality of discrete doses of a medicine in the form of a powder or a tablet. The elongated blister strip includes a base strip having a pocket/blister defined therein and a cover strip positioned on the base strip, where the base strip and the cover strip are peelably separated to allow access to content of each blister. Such a medicine delivery dispenser usually includes a mechanism for access to the doses, and the mechanism includes a peeling device for peeling the cover strip from the base strip. In this way, the medicine can be used to be delivered to a patient.

A suitable peeling device is positioned to peel the base strip and the cover strip of the blister sheet at an opening position of the device. The peeling device usually includes a (cover or base) strip driver, configured to pull a cover strip from a base strip of a blister received at an opening station. A strip driving device includes a wheel with a fixed diameter, where the cover strip is wound around the wheel. The wheel has an effective winding surface, and the diameter of the effective winding surface increases as more cover strip is wound around the wheel.

A problem encountered with using a wheel as a sheet driver for winding a medicine carrier sheet is that as the sheet is wound around the wheel, the effective winding diameter of the wheel increases, and therefore, an effective lateral pulling action (namely, a pulling force along the length of the sheet) also increases. This is problematic because it is desirable that upon actuation, the blisters on the medicine carrier experience a consistent pulling force at an open position to ensure that each blister of the medicine carrier experiences a substantially uniform indexing/opening effect. Generally, an insufficient pulling force results in the blister incapable of being opened because of misalignment or the inability to peel the cover strip, and an excessive pulling action causes stress on mechanical components and increases a force required for activating the delivery device.

The medicine delivery dispenser provided in this embodiment includes the rotation compensation mechanism 10 and a driving assembly. The rotation compensation mechanism 10 can compensate for any increase in the diameter of the effective winding surface of the wheel during use of the medicine delivery dispenser, to ensuring that the medicine carrier is evenly indexed during each time of activation of a delivery mechanism. The rotation compensation mechanism 10 is the rotation compensation mechanism 10 involved in any one of the foregoing embodiments. For specific structures and functions of the rotation compensation mechanism 10, refer to related descriptions of the rotation compensation mechanism 10 provided in the foregoing embodiments, and details are not described herein again. In this embodiment, the to-be-wound object can be a base strip or a cover strip.

The driving assembly is connected to the active rotation member 1, and configured to drive the active rotation member 1 to rotate. In a specific embodiment, the driving assembly includes a motor, preferably an electric motor. The electric motor can provide rotation drive. For example, the electric motor can include a DC motor, a piezoelectric (PZ) motor, an ultrasonic motor, a solenoid motor, or a linear motor. Preferably, an electronic driving system includes a DC motor, a PZ motor, or an ultrasonic motor. The driving assembly can alternatively be a manually-activated structure, for example, a transmission handle, and the like.

In a specific embodiment, the medicine delivery dispenser includes two rotation compensation mechanisms 10. One rotation compensation mechanism 10 rotates clockwise, and the other rotates anticlockwise. The medicine delivery dispenser can specifically accommodate two medicine carriers, and the two medicine carriers and the two rotation compensation mechanisms 10 are positioned in a one-to-one correspondence.

Specifically, the medicine delivery dispenser further includes an index wheel, an open station, and a base unit; and the index wheel, the open station, and the rotation compensation mechanism 10 are all positioned on a side surface of the base unit. The medicine carrier is a blister strip, and a plurality of pouches are positioned at intervals in the length direction of the blister strip. The two blister strips are positioned on the side of the two rotation compensation mechanisms 10 far from the edge of the medicine delivery dispenser to form a left cavity and a right cavity. In the medicine delivery dispenser, each blister strip cooperates with a respective index wheel, and successive pouches are therefore guided to a central open station. Two index wheels are randomly combined by rotation of a gear. At the open station, a base strip and a cover strip of each blister strip can be peeled away from the tip, an obtained empty base strip is wound in a respective receiving room, and an obtained cover strip is the foregoing to-be-wound object. One end of the to-be-wound object is fixed to a hook of the driven rotation member. The driving assembly provides a relatively stable driving pull force for each rotation compensation mechanism 10. In an operation process, the effective winding diameter of a cover strip on each driven rotation member gradually increases, and each rotation compensation mechanism 10 can provide driving compensation in the operation process.

The foregoing descriptions are merely implementations of this application, and the protection scope of this application is not limited thereto. All equivalent structure or process changes made according to the content of this specification and accompanying drawings in this application or by directly or indirectly applying this application in other related technical fields shall fall within the protection scope of this application.

## Claims

1. A rotation compensation mechanism, comprising:
an active rotation member connected to a driving assembly and rotatable under an action of the driving assembly;
a driven rotation member rotatably connected to the active rotation member and configured to wind a to-be-wound object; and
a damping assembly positioned between the active rotation member and the driven rotation member,
wherein the active rotation member applies driving torque to the driven rotation member through the damping assembly, and
wherein the driving torque is equal to or less than a preset threshold and the preset threshold is a maximum driving torque value where the active rotation member rotates relative to the driven rotation member.

2. The rotation compensation mechanism of claim 1, wherein the active rotation member applies same driving torque to the driven rotation member during clockwise rotation and counterclockwise rotation.

3. The rotation compensation mechanism of claim 1, wherein the damping assembly comprises an elastic damper that does not comprise a rigid material.

4. The rotation compensation mechanism of claim 1, wherein the damping assembly comprises one or more of an elastic material damper, a viscous grease damper, and a magnetic damper.

5. The rotation compensation mechanism of claim 4, wherein:
the active rotation member comprises a rotary shaft and a base plate;
the rotary shaft is vertically connected to a side surface of the base plate;
the driven rotation member comprises a hollow columnar body having a hole and is sleeved on the rotary shaft; and
the damping assembly is positioned between the base plate and an end surface of the hollow columnar body, and/or is positioned between an outer surface of the rotary shaft and an inner surface of the hollow columnar body.

6. The rotation compensation mechanism of claim 5, wherein:
the damping assembly comprises a first elastic material damper positioned between the base plate and the end surface of the hollow columnar body, and/or is positioned between the outer surface of the rotary shaft and the inner surface of the hollow columnar body; and
the first elastic material damper is in the shape of a closed loop.

7. The rotation compensation mechanism of claim 6, wherein the damping assembly further comprises:
at least one second elastic material damper spaced apart from the first elastic material damper and is positioned between the base plate and the end surface of the hollow columnar body and located on a side of the first elastic material damper proximate to the rotary shaft;
and/or
the at least one second elastic material damper is positioned between the outer surface of the rotary shaft and the inner surface of the hollow columnar body and located on the side of the first elastic material damper close to the base plate.

8. The rotation compensation mechanism of claim 7, wherein:
the at least one second elastic material damper is in the shape of a closed loop, and the first elastic material damper and the at least one second elastic material damper are coaxially sleeved; or
the at least one second elastic material damper is in a sheet shape and is spaced apart in the circumferential direction of the rotary shaft to form at least one ring shape, and the first elastic material damper and the at least one second elastic material damper are coaxially sleeved.

9. The rotation compensation mechanism of claim 8, wherein torque generated between the active rotation member and the driven rotation member by the first elastic material damper is less than torque generated between the active rotation member and the driven rotation member by the at least one second elastic material damper.

10. The rotation compensation mechanism of claim 9, further comprising a plurality of second elastic material dampers, and torque generated between the active rotation member and the driven rotation member by the plurality of second elastic material dampers gradually increases in the direction towards the rotary shaft.

11. The rotation compensation mechanism of any one of claims 6 to 10, wherein
the damping assembly comprises:
the elastic material damper,
a first engaging groove provided on a side surface of the active rotation member facing the driven rotation member, and/or a second engaging groove provided on a side surface of the driven rotation member facing the active rotation member;
the elastic material damper is partially elastically compressed in the first engaging groove and abuts against a bottom wall of the first engaging groove; and/or the elastic material damper is partially elastically compressed in the second engaging groove and abuts against a bottom wall of the second engaging groove; and
the driven rotation member is attached to a surface of the active rotation member.

12. The rotation compensation mechanism of claim 5, wherein
the damping assembly comprises the viscous grease damper; and the viscous grease damper comprises an annular viscous grease damper positioned between the base plate and the end surface of the hollow columnar body, and/or
the viscous grease damper comprises a hollow columnar viscous grease damper positioned between the outer surface of the rotary shaft and the inner surface of the hollow columnar body.

13. The rotation compensation mechanism of claim 12, further comprising:
a first sealing member and a second sealing member, both being clamped between the active rotation member and the driven rotation member,
wherein the viscous grease damper comprises the annular viscous grease damper positioned between the base plate and the end surface of the hollow columnar body, and the first sealing member and the second sealing member are respectively positioned on the outer side and the inner side of the annular viscous grease damper; and/or
wherein the viscous grease damper comprises the hollow columnar viscous grease damper positioned between the outer surface of the rotary shaft and the inner surface of the hollow columnar body, and the first sealing member and the second sealing member are respectively positioned at two opposite ends of the hollow columnar viscous grease damper.

14. The rotation compensation mechanism of claim 4, wherein the damping assembly comprises a magnetic damping assembly comprising:
a first magnetic member and a second magnetic member that are oppositely positioned, wherein one of the first magnetic member and the second magnetic member is positioned on a side of the driven rotation member facing the active rotation member, the other is positioned on a side of the active rotation member facing the driven rotation member, and the first magnetic member and the second magnetic member are attracted to each other by magnetism to generate torque between the first magnetic member and the second magnetic member.

15. A medicine delivery dispenser, comprising a rotation compensation mechanism according to any one of claims 1 to 14; and
a driving assembly connected to the active rotation member and being configured to drive the active rotation member to rotate.
